# EUROPEAN PATENT APPLICATION

(11) **EP 2 937 799 A1**
(43) Date of publication of application: **28.10.2015**
(21) Application number: 14165421.0
(22) Date of filing: 22.04.2014
(51) Int. Cl.: G06F 19/00

(54) **Self adapting clinical information timeline**

(71) Applicant: Agfa HealthCare, 53227 Bonn (DE)
(72) Inventor: Mouluquet, Laurent, 2640 Mortsel (BE); Alard, Yohan, 2640 Mortsel (BE); Ziade, Olivier, 2640 Mortsel (BE)
(74) Representative: Verbrugghe, Anne Marie L.

(57) **Abstract**

A computer-implemented method for adapting a timeline to a zoom command carried out by a user, in a medical information system, comprising the steps of:
- displaying the timeline with individual markers representing discrete clinical events,
- in a processing module interpreting and processing a zoom command effectuated by the user to determine whether a predefined zoom factor retrieved from a memory is exceeded, depending the case, said processing module controlling display of said timeline either:
- as a summary bar containing a full text clinical summary and where the width of the bar represents the duration of the clinical event or,
- as an update of the original timeline view where the displayed markers are adjusted according to the changed zoom factor and an updated timeline center.

## Description

### FIELD OF THE INVENTION

The present invention relates generally to the management of clinical information by means of charts of timelines which are being created and maintained for use in a clinical environment.

### BACKGROUND OF THE INVENTION

In clinical environments (such as hospitals, private practices, veterinary practices, or others) a lot of clinical information has to be dealt with by the medical staff. It is not uncommon nowadays that clinical information is being dealt with in electronic format, and that clinical information is entered and afterwards interpreted on a computer display. An important advantage of this is that more information can be interpreted quicker and more effectively by the users through the use of graphical representations of data.

The prescription and administration of a medicine is an example of a common, but important, clinical procedure. It is the manner in which a medicine is administered that will determine to some extent whether or not the patient gains any clinical benefit, and whether they suffer any adverse effect from their medicines.

In a hospital in-patient setting, it is a complex aspect of the daily occupation of the staff to maintain overview on the different treatment schedules of the different patients. In the prior art, written templates have been used to create and maintain these medical charts for the patients during their treatment in the hospital. These written charts are used to keep track of the prescriptions and the delivery of the medication to the patient. The doctor will instruct the staff by means of a written (and signed) instruction about a prescription to be delivered to the patient.

However, these written charts are not dynamic and are not very easy to adjust or correct. It has been described in the prior art that nowadays these charts are replaced by electronic equivalents which are processed, stored and displayed in so-called medication management systems, generally known as part of a hospital information system.

Especially, the display of a drug prescription on a timeline is a good example of a popular and comprehensive way of presenting a complex set of information to the user. The advantages of electronically generated and displayed timelines are that the characteristics of the view of the timeline can be adapted by the user to his own needs. Under certain circumstances the user may require the detailed information of a single element of the medication prescription (e.g. the exact time of administration of a dosage, accompanied with the dosage parameters), where alternatively, the user could be interested in an overview of the complete prescription period (e.g. in order to evaluate or compare the said prescription to other elements of the patients' treatment or to evaluate the clinical responses of measurements or observations in relation to the said prescription).

The most important characteristic of a timeline view is the scale at which the timeline is displayed. The zoom-factor which is associated with this scale determines the span or width of the visible time period depicted by the timeline. Another characteristic of a timeline view can be the level of detail of the data (or the granularity of the displayed information). In our example, we will limit ourselves to the zoom-factor as the main characteristic considered.

The most common way of representing a medication prescription on a timeline is to depict the different dosage events as individual discrete events on the timeline, where each element (most commonly presented as a marker) is accompanied with some more detailed information related to the individual event and is most commonly shown as text labels appearing next to the markers.

This leads to a situation where, when the timeline is being zoomed out, many individual events have to be shown in a small area of the available timeline space (on the computer display which has a limited surface) so that the timeline may become cluttered. This cluttering may manifest itself in the fact that the individual markers (and/or their related text labels) overlap with the neighbouring markers, which renders the exact position of the markers insufficiently accurate or which makes the text in the displayed labels unreadable for some or all of the markers on the timeline.

### SUMMARY OF THE INVENTION

The present invention provides a computer-implemented method for adapting a timeline to a zoom command carried out by a user, in a medical information system, comprising the steps of:
- displaying the timeline with individual markers representing discrete clinical events,
- in a processing module interpreting and processing a zoom command effectuated by the user to determine whether a predefined zoom factor retrieved from a memory is exceeded, depending the case, said processing module controlling display of said timeline either:
- as a summary bar containing a full text clinical summary and where the width of the bar represents the duration of the clinical event or,
- as an update of the original timeline view where the displayed markers are adjusted according to the changed zoom factor and an updated timeline center.

In the context of this invention, the zoom operation is the trigger for the method described in the invention. This zoom operation has to be understood as any method which is implemented on a computer device to capture the parameters unambiguously defining a zoom operation on a timeline displayed on a computer display.

The zoom operation is unambiguously characterized by 2 parameters: a zoom factor and zoom center. The zoom factor is a number which determines by how much the current view is enlarged relatively to a starting point or reference view. A zoom factor equal to "1" represents no scaling or zooming, and results in the display of the reference view. A negative zoom factor results in a scaling down of the view compared to the reference view. The zoom center determines the center point of the timeline over which the visible zoomed timeline area is spread out equally. Many zoom centers may be possible resulting in the display of the same timeline at a same magnification, but showing a different range of the timeline.

The zoom operation is in practice carried out by applying movement or gesture (or combination of both) on an input device such as a computer mouse (e.g. through application of the scroll wheel, or through "dragging", i.e. moving the mouse while keeping one of the buttons depressed,...), or a touch screen display, or by manipulating a slider on a screen. In the context of this invention, the zoom operation has to be understood as the operation delivering the input for the processing module to start its calculations. Various methods for performing the zoom operation using different devices are described in the art.

Subsequently, the processing module will use the zoom factor value to evaluate in a first instance whether or not a stored threshold value is exceeded. The threshold value being the zoom factor at which it is desirable to display the sequence of clinical events as a bar-like graph containing a summarizing text describing the clinical prescription, procedure or treatment. The zoom factor and zoom center value will be used in combination to further recalculate the new timeline view characteristics after the zoom operation.

The processing module may include dedicated hardware, software and/or firmware for performing information processing. The processing module can be connected to a memory, and can be capable of reading in data from the said memory and from input devices connected to the processor (such as a touch screen, a keyboard, a mouse, a bar code scanner, ...). The processor can also be connected to output devices, such as a printer, a computer screen or monitor, an external memory for rendering or storing the timeline chart.

The present invention can be implemented as a computer program product adapted to carry out the steps set out in the description. The computer executable program code adapted to carry out the steps set out in the description can be stored on a computer readable medium.

The present invention is beneficial in that the aforementioned data cluttering on the display can be avoided in order to ensure that relevant and clearly readable information is available under all viewing circumstances: zoomed in, or zoomed out.

Further advantages and embodiments of the present invention will become apparent from the following description and drawings.

### BRIEF DESCRIPTION OF THE DRAWINGS

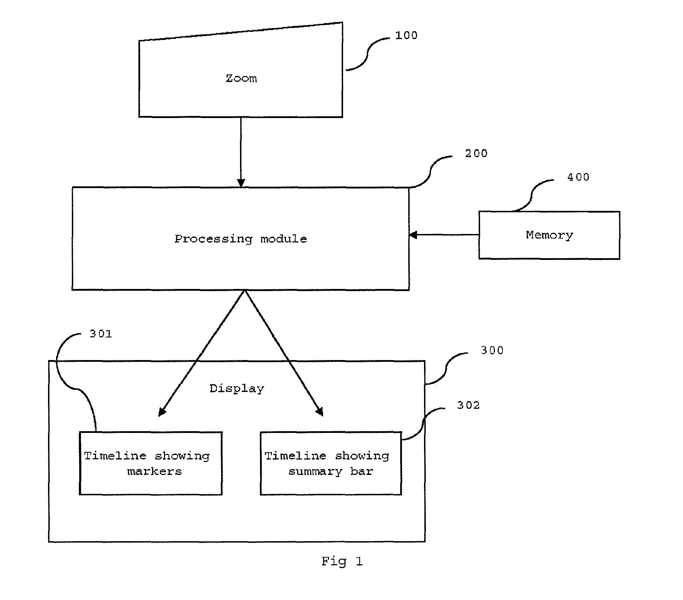

Fig. 1 shows a flowchart illustrating a method for adapting a timeline to a zoom command carried out by a user, in a medical information system, consisting of the zoom operation [100], the processing module [200], a memory [400] and a display showing a timeline [300]. The display [300] is capable to display 2 types of timelines [301] and [302].

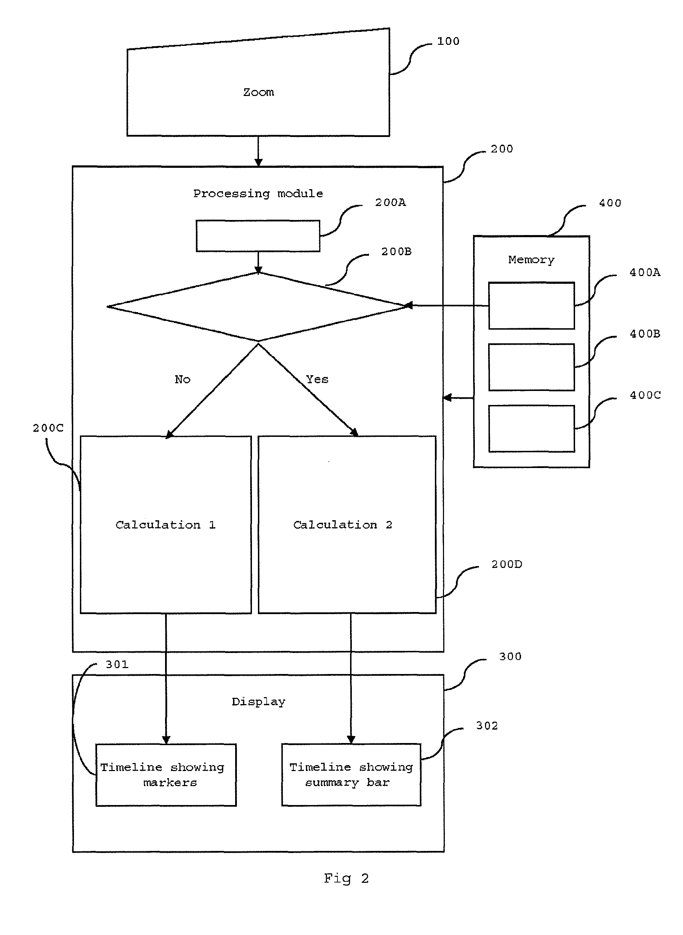

Fig 2. shows the same flowchart as in Fig. 1 except for that the functioning of the different modules is explained in more detail. Figure 2 reveals the working of the processing module [200] and the way the stored data in the memory [400] is used.

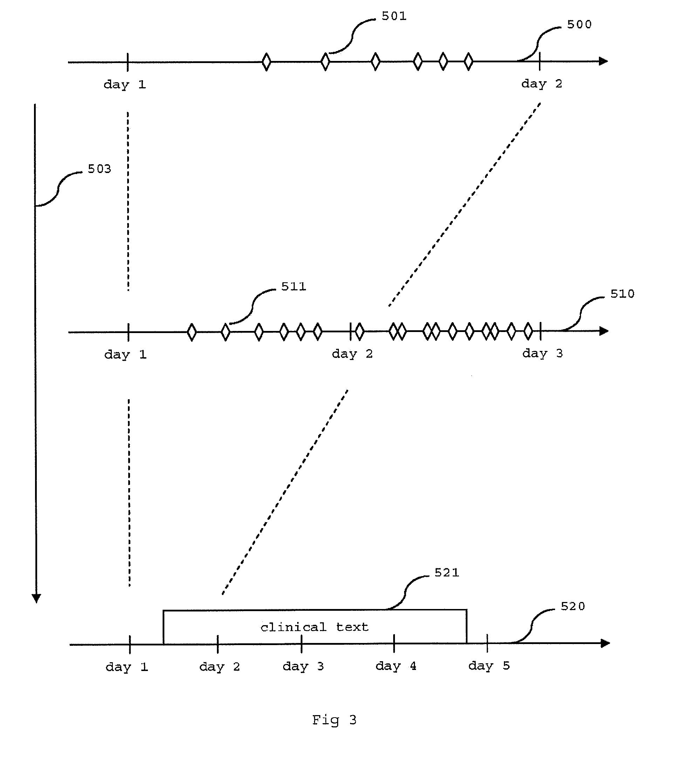

Fig 3. shows 3 embodiments of the same timeline [500, 510 and 520], each displayed at different zoom factors. The direction of the arrow [503] indicates the direction of a "zooming out" operation. The effect of the zoom operation on the individual markers [501 and 511] is shown on their respective timelines [500 and 510]. Timeline [520] again shows the same timeline, but now further zoomed out and thus depicted as a summary bar [521] containing a text summary.

### DETAILED DESCRIPTION OF THE INVENTION

In the following detailed description, reference is made in sufficient detail to the above referenced drawings, allowing those skilled in the art to practice the embodiments explained below.

Embodiments of the present invention provide a method for adapting a timeline to a zoom command carried out by a user, in a medical information system. The medical information management system is typically implemented on a computer system comprising a means for inputting data (such as a keyboard, a touch screen, a computer mouse, a bar code scanner or other means), a means for storing the medical data (such as a computer memory), a processor for applying calculations, rules and comparisons on the data, and a means of displaying or storing the results (in the shape of for instance the medication chart) such as a computer monitor, a printer, and/or an external computer memory.
Fig. 1 represents a block diagram illustrating the method of adapting a timeline to a zoom command carried out by a user as described in an embodiment of the invention. As a first step, the zoom operation [100] is carried out using an input device such as a mouse or a touch screen, or any other method described in the art to perform a zoom command on a scalable object. The zoom operation collects and passes on updated values for the zoom factor and zoom center to the processing module [200]. The processing module performs evaluations on the updated data and the stored information in memory [400] in order to decide which type of timeline [300] will be displayed - [301] or [302]-, and in order to calculate the data to be displayed on the timeline.
Fig. 2 represents a block diagram illustrating the same method as in Fig 1, providing further detailed information on the functioning of the processing module [200] in the context of this invention. The trigger for the processing module [200] to start it's operation is when it receives new data coming from the zoom operation input device. The input device will supply to the processing module raw parameters which are translated into numeric values for zoom and the mouse cursor coordinates. This readout is handled at the computer system level (managed by the operating system of the computer, and processed by the so-called device driver of the input device). For the context of this invention, it is not relevant to describe the different types of mechanisms and techniques, which result in the read-out of the input device data.

New values for the zoom factor and the zoom center are calculated in the processing module [200A] from these input data as a result of the zoom operation [100] effectuated by the user.

As explained above, the zoom operation is in practice carried out by applying movement or gesture (or combination of both) on an input device such as a computer mouse, a touch screen display, or by manipulating a slider on a screen. The amount of zoom applied translates into the updated zoom factor. The updated value of the zoom center is derived from the same user input operation as explained above, but contains the location information of where on the timeline the center of the zoom operation is assumed to take place.

The processing module will calculate the relative increase/decrease of the zoom-factor in comparison to the actual applied value in the current display view, and will evaluate [200B] whether the updated zoom factor exceeds the stored zoom factor threshold in memory [400A] or not.

In the case that the updated zoom factor obtained from the zoom operation does not exceed the zoom factor threshold [400A] stored in memory, the timeline still will be recalculated using Calculation 1 [200C] to such effect that the relative increase/decrease of zoom is reflected both in an updated timeline axis and an update of the respective markers which represent the clinical events within the visible area of the displayed timeline [301]. The markers to be displayed on the visible area of the timeline are retrieved from memory [400B].

In the alternate case that the updated zoom factor obtained from the zoom operation does exceed the zoom factor threshold [400A] stored in memory, the timeline will be recalculated using Calculation 2 [200D] to such effect that the relative increase/decrease of zoom is reflected in an updated timeline axis, and that the series of clinical events will be summarized in a summary bar containing the clinical description of the series of events in a text summary.

In one embodiment, the timeline is a medication prescription timeline. Other embodiments of the timeline may apply to other treatment timelines (such as timelines for dialysis, surgery, or other) or display of clinical measurement events on a timeline.

In Fig 3, the effect of a zoom command on an embodiment of a timeline is shown. The direction of the arrow [503] indicates the direction of a "zooming out" operation (in which case the zoomfactor decreases). The timeline [500] shows the clinical events as individual markers [501] on the timeline. The effect of the zoom operation [503] is shown on timeline [510], which shows in comparison to timeline [500] a larger time period represented on the same timeline space. The scale of the timeline has changed as such that a timespan of 2 days is now displayed (in comparison with timeline [500]). The consequence of this operation is that the number of markers [511] displayed on the timeline has increased, and the information is thus denser.
Timeline [520] shows the effect of a further "zooming out" operation causing the threshold zoomfactor to be exceeded by the updated zoomfactor, with the consequence that the series of clinical events is summarized in a summary bar [521] containing the clinical description of the series of events in a text summary. The scale of the timeline in this example has further changed showing now a timespan of 5 days in total.

## Claims

1. A computer-implemented method for adapting a timeline to a zoom command carried out by a user, in a medical information system, comprising the steps of:
- displaying the timeline with individual markers representing discrete clinical events,
- in a processing module interpreting and processing a zoom command effectuated by the user to determine whether a predefined zoom factor retrieved from a memory is exceeded, depending the case, said processing module controlling display of said timeline either:
- as a summary bar containing a full text clinical summary and where the width of the bar represents the duration of the clinical event or,
- as an update of the original timeline view where the displayed markers are adjusted according to the changed zoom factor and an updated timeline center.
